# EUROPEAN PATENT APPLICATION

(11) **EP 2 728 356 A1**
(43) Date of publication of application: **07.05.2014**
(21) Application number: 12804364.3
(22) Date of filing: 29.06.2012
(51) Int. Cl.: G01N 33/543, G01N 33/553

(54) **CONJUGATE FOR USE IN IMMUNOASSAY METHOD**

(30) Priority: 30.06.2011 JP 2011146604
(71) Applicant: Sekisui Medical Co., Ltd., Tokyo 103-0027 (JP)
(72) Inventor: YOKOKAWA, Shinya, Ryugasaki-shi Ibaraki 301-0852 (JP); YAMAMOTO, Mitsuaki, Ryugasaki-shi Ibaraki 301-0852 (JP); SHIMIZU, Tomo, Ryugasaki-shi Ibaraki 301-0852 (JP)
(74) Representative: HOFFMANN EITLE
(86) International application number: PCT/JP2012/066816
(87) International publication number: WO 2013/002403

(57) **Abstract**

To adjust detection sensitivity and expand a measurement range depending on objects to be detected by a simple procedure in an immunoassay method involving using a colloidal metal having immobilized thereon an antibody or an antigen. Blocking treatment for a surface of a colloidal metal particle having immobilized thereon an antibody or an antigen with both components, i.e., a component of biological origin and a component of non-biological origin enables adjustment of measurement sensitivity with, for example, kinds and concentrations of both the components, and enables use of the component of non-biological origin, which has not been usable alone because of aggregation of a colloidal metal.

## Description

### Technical Field

The present invention relates to a conjugate including a colloidal metal sensitized with an antibody or an antigen, for use in an immunoassay method such as immunochromatography (hereinafter sometimes simply referred to as IC). In particular, the present invention relates to a conjugate blocked with a component of non-biological origin and a component of biological origin. The present invention also relates to an IC detection method and a test strip for immunochromatography each using the conjugate, and a manufacturing method for the conjugate.

### Background Art

An example of a label that serves as a component of a conjugate, such as a labeled antibody, for use in an immunoassay method is a colloidal gold particle (hereinafter sometimes simply referred to as colloidal gold), and coloring caused by the colloidal gold particle enables confirmation of a measurement result. Further, on the basis of a degree of the coloring, it is also possible to quantitatively detect a substance to be detected in a sample.

When the colloidal gold is used as the label that serves as a component of the labeled antibody, a surface of the colloidal gold has property of being liable to non-specifically adsorb proteins and other substances derived from the sample as well as the intentionally bound antibody. To suppress such non-specific adsorption, there is generally performed blocking treatment involving coating part of the surface of the colloidal gold to which the antibody is not bound with a blocking agent of biological origin containing bovine serum albumin (BSA), casein, or the like as an active component (hereinafter sometimes simply referred to as blocking agent of biological origin). A blocking agent containing a component of non-biological origin as an active component (hereinafter sometimes simply referred to as blocking agent of non-biological origin) is also commercially available. However, when the inventors of the present invention simply replaced the blocking agent of biological origin with the blocking agent of non-biological origin and used the latter alone, the conjugate itself aggregated after the blocking treatment, and hence was unable to be resuspended, or the blocking agent of non-biological origin tended to naturally aggregate, and hence was impaired in stability as a reagent, for example. Consequently, the inventors were unable to find any commercially available blocking agent of non-biological origin capable of being put into practical use.

On the other hand, in quantitative immunoassay using a labeled antibody, when the substance to be detected is a multivalent antigen, the following problem arises. A measurement signal rapidly increases along with an increase in antigen concentration due to sequential crosslinking of antigens via the labeled antibody, and thus a difference in sensitivity depending on concentration of the substance to be detected is not observed in a range in which the antigen concentration is high, with the result that accurate quantification can not be performed. Inthiscase, itisnotpreferred to perform an operation of diluting a specimen because a measurement error becomes liable to occur and the procedure is complicated by addition of a step for the diluting operation. In the first place, prediction of which specimen has a high antigen concentration is not practical itself.

As a technology for expanding a concentration range in which a substance to be detected can be measured, for example, there is given a technology described in Patent Literature 1. In Patent Literature 1, there is a disclosure of a technology involving using, as a conjugate, a mixture of at least two kinds of colloidal gold particle groups having different particle size distributions, in a test strip for immunochromatography including a labeled antibody obtained by immobilizing an antibody on colloidal gold. However, preparation of many kinds of mixtures of colloidal gold having different particle size distributions depending on substances to be detected and purposes is not practical because of the time and cost required.

### Related Art Documents

### Patent Documents

Patent Document 1: JP 2010-32396 A

### Summary of Invention

### Problem to be solved by the invention

In order to solve the problems of the related art, an obj ect to be achieved by the present invention is to adjust detection sensitivity and expand a measurement range depending on objects to be detected by a simple method in the case of performing immunoassay using a colloidal metal having immobilized thereon an antibody or an antigen, while suppressing aggregation between conjugates after blocking treatment. Another object is to prevent the non-specific adsorptionof impurities and the like onto the surface of the colloidal metal while achieving the above-mentioned object.

### Means of solving the problem

The inventors of the present invention have made intensive studies in order to achieve such objects, and as a result, have found that the use of a component of non-biological origin in combination with a component of biological origin in the blocking of the surface of a colloidal metal particle having immobilized thereon an antibody enables the adjustment of measurement sensitivity and/or the expansion of a measurement range. Thus, the inventors have completed the present invention. That is, whereas, hitherto, it has been impossible to use the component of non-biological origin as a blocking agent because the colloidal metal aggregates when the component of non-biological origin is used alone as a blocking agent, the inventors have for the first time found that its combined use with the component of biological origin not only enables the use as a blocking agent but also exhibits novel effects, i.e., the adjustment of detection sensitivity and/or the expansion of a measurement range.

Specifically, the present invention has the following constructions.
<1> A conjugate, including a colloidal metal having a surface sensitized with an antibody or an antigen, in which the surface of the colloidal metal is blocked with a component of non-biological origin and a component of biological origin.
<2> The conjugate according to the construction <1>, in which the component of biological origin includes one or more members selected from the group consisting of: BSA; casein; sericin; a polypeptide corresponding to amino acids 419 to 607 of an amino acid sequence of DnaK, which is a heat shock protein (HSP) of *Escherichia coli* origin; NEO PROTEIN SAVER; StartingBlock™ (PBS) Blocking Buffer; and Stabil Coat.
<3> The conjugate according to the construction <1> or <2>, in which the component of non-biological origin includes one or more members selected from the group consisting of: a methacrylic acid polyoxyalkylene ester; NB4025; SN100; StabilGuard; and Protein-Free Blocking Buffer.
<4> The conjugate according to any one of the constructions <1> to <3>, in which the methacrylic acid polyoxyalkylene ester includes one or more members selected from the group consisting of: polyoxyethylene monomethacrylate; polyoxyethylene polyoxytetramethylene monomethacrylate; polyoxyethylene polyoxypropylene monomethacrylate; and octylpolyoxyethylene polyoxypropylene monomethacrylate.
<5> The conjugate according to any one of the constructions <1> to<4>, in which the colloidal metal is colloidal platinum or colloidal gold.
<6> A detection method for a substance to be detected in a sample, the detection method including bringing the sample into contact with a conjugate including a colloidal metal having a surface sensitized with an antibody or an antigen, thesurfaceof the colloidal metal being blocked with a component of non-biological origin and a component of biological origin.
<7> The detection method according to the construction <6>, in which the conjugate is carried on a test strip for immunochromatography.
<8> The detection method according to the construction <6> or <7>, in which the component of biological origin includes one or more members selected from the group consisting of: BSA; casein; sericin; a polypeptide corresponding to amino acids 419 to 607 of an amino acid sequence of DnaK, which is a heat shock protein (HSP) of *Escherichia coli* origin; NEO PROTEIN SAVER; StartingBlock™ (PBS) Blocking Buffer; and Stabil Coat.
<9> The detection method according to any one of the constructions <6> to <8>, in which the component of non-biological origin includes one or more members selected from the group consisting of: a methacrylic acid polyoxyalkylene ester; NB4025; SN100; Stabil Guard; and Protein-Free Blocking Buffer.
<10> The detection method according to any one of the constructions <6> to <9>, in which the methacrylic acid polyoxyalkylene ester includes one or more members selected from the group consisting of: polyoxyethylene monomethacrylate; polyoxyethylene polyoxytetramethylene monomethacrylate; polyoxyethylene polyoxypropylene monomethacrylate; and octylpolyoxyethylene polyoxypropylene monomethacrylate.
<11> The detection method according to any one of the constructions <6> to <10>, in which the substance to be detected includes a multivalent antigen.
<12> The detection method according to the construction <11>, in which the substance to be detected includes a D-dimer.
<13> The detection method according to any one of the constructions <6> to <12>, in which the colloidal metal is colloidal platinum or colloidal gold.
<14> A test strip for immunochromatography, including the following components:
   (1) a conjugate pad that carries a conjugate including a colloidal metal having a surface sensitized with a first antibody or an antigen, the surface of the colloidal metal being blocked with a component of non-biological origin and a component of biological origin; and
   (2) an insoluble membrane that includes a second antibody that binds to a complex of the conjugate and a substance to be detected, the insoluble membrane being disposed downstream of the conjugate pad as the component (1).
<15> The test strip according to the construction <14>, in which the colloidal metal is colloidal platinum or colloidal gold.
<16> The detection device, including: the test strip for immunochromatography according to the construction <14> or <15>; and a housing.
<17> A manufacturing method for a conjugate including a colloidal metal sensitized with an antibody or an antigen, the manufacturing method including the following steps:
   (a) a step of sensitizing a colloidal metal with an antibody or an antigen to provide a conjugate;
   (b) a step of subjecting a surface of the colloidal metal to blocking treatment by bringing the conjugate into contact with a component of non-biological origin and a component of biological origin; and
   (c) a step of concentrating the conjugate subjected to the blocking treatment by centrifugation.
<18> The manufacturing method for a conjugate according to the construction <17>, in which the component of biological origin includes one or more members selected from the group consisting of: BSA; casein; sericin; a polypeptide corresponding to amino acids 419 to 607 of an amino acid sequence of DnaK, which is a heat shock protein (HSP) of *Escherichia coli* origin; NEO PROTEIN SAVER; StartingBlock™ (PBS) Blocking Buffer; and Stabil Coat.
<19> The manufacturing method for a conjugate according to the construction <17> or <18>, in which the component of non-biological origin includes one or more members selected from the group consisting of: a methacrylic acid polyoxyalkylene ester; NB4025; SN100; Stabil Guard; and Protein-Free Blocking Buffer.
<20> The manufacturing method according to the construction <17>, in which the step (b) includes a step of subjecting the surface of the colloidal metal to the blocking treatment by bringing the conjugate into contact with the component of non-biological origin, and then bringing the conjugate into contact with the component of biological origin.
<21> The manufacturing method according to any one of the constructions <17> to <20>, in which the colloidal metal is colloidal platinum or colloidal gold.

### Effects of the Invention

In an immunoassay method involving using a conjugate including a colloidal metal having immobilized thereon an antibody or an antigen, whereas, hitherto, the use of a blocking agent of non-biological origin alone for blocking has caused aggregation between colloidal metal particles and the resultant has not been usable as the conjugate, the use as the conjugate is enabled by blocking the surface of the colloidal metal particle with both the component of biological origin and the component of non-biological origin. Further, the blocking of the surface of the colloidal metal particle with both the component of biological origin and the component of non-biological origin enables the adjustment of sensitivity or the expansion of a measurement range depending on objects to be detected. Further, the blocking of the surface of the colloidal metal particle with both the component of biological origin and the component of non-biological origin enables the reaction behaviors of a reference substance for concentration calibration and a substance to be detected in a specimen to be identical to each other, and enables improvements in quantitativeness and correlation with a reference measurement method.

By virtue of the enablement of the above-mentioned effects, for example, a test strip can be universally designed so as to be fit for the detection performance of a detector, and the simultaneous detection of two or more substances to be detected in different concentration regions with one sample and one test strip is facilitated. Further, a desired conjugate can be manufactured through an easier operation than ever before, resulting in good economic efficiency. The present invention can also meet a wide range of needs in the field of point-of-care (POC) testing.

### Brief Description of Drawings

FIG. 1 is a schematic structural view of a test strip of the present invention.
FIGS. 2 are graphs showing comparison of measurement sensitivity depending on antigen concentrations in the cases of blocking colloidal gold with each of formulations according to the present invention and a formulation of BSA alone.
FIGS. 3 are graphs showing comparison of measurement sensitivity depending on antigen concentrations in the cases of blocking colloidal gold with each of formulations according to the present invention and a formulation of BPF alone.
FIG. 4 is a graph showing the results of a study on the addition order of blocking agents.
FIG. 5 is a graph showing the results of a study on usable concentration ranges for various blocking agents.

### Embodiments of the invention

### (Conjugate)

A conjugate of the present invention includes a colloidal metal sensitized with (having immobilized thereon) a substance that specifically binds to a substance to be detected in a sample, such as an antibody or an antigen, the conjugate being characterized in that the surface of the colloidal metal is blocked with a component of non-biological origin and a component of biological origin.

The conjugate of the present invention may be used in a measurement method based on an antigen-antibody reaction between an antibody or antigen immobilized on a particle surface and a substance to be detected in a specimen, such as an IC method or a turbidimetric immunoassay method (TIA method). In the following description, an IC method in which an antibody is immobilized on a membrane is taken as an example. A person skilled in the art can easily understand that, when the substance to be detected is an antibody, it suffices to immobilize a substance capable of capturing the antibody (an antigen, or an antibody against the antibody serving as the object to be detected) on a colloidal metal or an insoluble membrane. In the following description, the case where the substance that specifically binds to the substance to be detected in the sample is an antibody or an antigen is taken as an example.

### (Colloidal metal)

The colloidal metal to be used in the present invention may be any colloidal metal as long as the colloidal metal can construct the conjugate by being sensitized with an antibody or an antigen and can play a role as a label in a detection method for a substance to be detected (an antigen or an antibody) in a sample by being brought into contact with the sample.

The colloidal metal may be, for example, colloidal gold, colloidal platinum, colloidal silver, colloidal palladium, colloidal copper, colloidal nickel, colloidal indium, oracomposite colloid thereof. Of those, colloidal gold or colloidal platinum is suitable. In addition, the colloidal metal as referred to herein also encompasses a fine particle having a surface processed with a metal, such as a fine particle in which a non-metal particle such as a latex particle or a silica particle serves as a core particle and its surface is coated with gold.

Hereinafter, the present invention is described by taking the colloidal gold as a typical example of the colloidal metal. The particle diameter of the colloidal gold is known to greatly affect detection sensitivity. For example, when the conjugate is used by being carried on a test strip for immunochromatography, the particle diameter of the colloidal gold is preferably 20 to 60 nm, more preferably 30 to 50 nm, particularly preferably 30 nm. The colloidal gold may be manufactured by a generally known method involving, for example, adding an aqueous solution of trisodium citrate or an aqueous solution of triammonium citrate dropwise to a heated aqueous solution of tetrachloroauric (III) acid under stirring. The colloidal gold is herein sometimes referred to as colloidal gold particle, and these terms have the same meaning.

### (Immobilization of antibody or antigen on colloidal gold)

The immobilization of the antibody against the object to be detected or of the antigen on the colloidal gold is generally performed through physical adsorption in a solution obtained by mixing the colloidal gold and the antibody or the antigen. When the immobilization of the antibody is described as an example, the concentration of the antibody is preferably adjusted to 1 µg/mL to 5 µg/mL, and a buffer and its pH are preferably a 10 mmol/L phosphate buffer (pH 6 to 7) or a 10 mmol/L Tris-HCl buffer (pH 7 to 9), more preferably a 10 mmol/L Tris-HCl buffer (pH 7.5), but are not limited thereto, including the use of other buffers. Herein, not only the colloidal gold having immobilized thereon the antibody against the object to be detected or the antigen as described above, but also one having immobilized thereon a control antibody is referred to as "conjugate".

### (Blocking)

The conjugate of the present invention is characterized in that a region in the surface of the colloidal gold to which the antibody is not bound is blocked with both the component of non-biological origin and the component of biological origin.

Hitherto, for the blocking of colloidal gold, a protein of biological origin such as BSA, casein, or gelatin has been generally solely used, and a blocking agent of non-biological origin such as one used for other carriers such as latex particles has not been used for the colloidal gold. This is because the use of the blocking agent of non-biological origin for the blocking of the colloidal gold may cause non-specific aggregation of the colloidal gold. Under such circumstances, the inventors of the present invention have used both the component of non-biological origin and the component of biological origin as blocking agents for the colloidal gold to block the surface of the colloidal gold with the component of non-biological origin and the component of biological origin, and thus succeeded in adjusting detection sensitivity and expanding a measurement range.

That is, desired measurement depending on objects to be detected can be performed by investigating in advance the measurement sensitivity and the measurement range for each combination of a component of non-biological origin and a component of biological origin for each object to be detected and selecting the combination depending on purposes.

It should be noted that, herein, the "adjustment of detection sensitivity" refers to increasing or decreasing a measurement value as compared to the case of measurement of a substance to be detected having a certain concentration in a certain measurement system by a general technique. In addition, the "expansion of a measurement range" refers to expanding a range in which a substance to be detected in a specimen can be measured in a concentration-dependent manner in a low-concentration or high-concentration region, as compared to a conventional method.

Without wishing to be bound by any particular theory, it is conceivable that the component of biological origin and the component of non-biological origin have different tendencies in adsorption onto the surface of the colloidal metal, and hence are in such a relationship that both the components mutually fill gaps. In addition, it is also predicted that both the components adsorbed on the surface control a three-dimensional relationship among, for example, the antibody immobilized on the metal surface and various non-specifically adsorbed substances. It is estimated that one or more of those mechanisms function to enable the adjustment of detection sensitivity and the expansion of a measurement range depending on the combination of the respective components.

### (Component of non-biological origin)

The component of non-biological origin to be used for the blocking of the present invention may be any component as long as the component is not derived from an organism and has a blocking action. An example thereof is a methacrylic acid polyoxyalkylene ester. Specific examples thereof include: synthetic polymers such as polyoxyethylene monomethacrylate; polyoxyethylene polyoxytetramethylene monomethacrylate; polyoxyethylene polyoxypropylene monomethacrylate; and octylpolyoxyethylene polyoxypropylene monomethacrylate. More specific examples thereof include commercially available blocking agents such as N101 (containing polyoxyethylene polyoxytetramethylene monomethacrylate as a main component); N102 (containing polyoxyethylene polyoxypropylene monomethacrylate as a main component); SN100 (all of which are manufacturedbyNOF CORPORATION); NB4025 (manufactured by COSMO BIO CO., LTD.); Stabil Guard (manufactured by SurModics Inc.) ; and Protein-Free Blocking Buffer (manufactured by Thermo Fisher Scientific Inc.). Of those, N101, N102, and NB4025 are preferred.

In addition to the examples above, the blocking agent of non-biological origin that may be used in the present invention may be selected by a test method described in Test Example 1 and Test Example 2 to be described later.

Aconcentrationat which the component of non-biological origin is used has only to be appropriately determined depending on the component to be used. For example, the blocking may be performed by adding and mixing an antibody solution in a colloidal gold solution adjusted to 1 OD/mL, and then adding the above-mentioned commercially available product to the mixed solution so as to have a final concentration of from 0.1 to 15% when the concentration of the undiluted blocking agent is defined as 100%. Further, the lower limit of the final concentration is not limited to 0.1 % so that any numerical number such as 0.2, 0.3, 0.4, 0.5, 0.6, 0.7, 0.8, 0.9, or 1% maybe selected as the lower limit of the final concentration. In addition, the upper limit of the final concentration is not limited to 15 % so that any numerical number such as 14, 13, 12, 11, or 10% may be selected as the upper limit of the final concentration. In addition, their combination is, for example, any combination of the above-mentioned upper limits and lower limits, such as 0.2 to 14, 0.3 to 13, 0.4 to 12, 0.5 to 11, 0.6 to 10, 0.7 to 9, 0.8 to 8, 0.9 to 7, or 1 to 6%. Of those, a final concentration of from 0.5 to 10% may be preferably used.

The component of non-biological origin may be obtained by chemical synthesis, and can be quantified by an appropriate quantification method depending on its structure, such as quantification by titration of a carboxy group derived from methacrylic acid in the case of a methacrylic acid polyoxyalkylene ester, quantification of a vinyl group by NMR, or quantification based on molecular weight measurement by MS or GPC.

### (Component of biological origin)

The component of biological origin to be used for the blocking of the present invention may be any component as long as the component is derived from an organism and has a blocking action. Examples thereof include: animal or plant proteins; and peptides derived from animal or plant proteins. Specific examples thereof include: BSA, which stands for bovine serum albumin; casein; Blocking Peptide Fragment (BPF: manufactured by TOYOBO), which is a polypeptide corresponding to amino acids 419 to 607 of the amino acid sequence of DnaK, which is a heat shock protein (HSP) of *Escherichia coli* origin; NEO PROTEIN SAVER (manufactured by TOYOBO) of silk protein origin (hydrolysate of sericin); StartingBlock™ (PBS) Blocking Buffer (manufactured by PIERCE); and Stabil Coat (manufactured by SurModics, Inc.).

The concentration of the component of biological origin has only to be appropriately determined depending on the component to be used. For example, the blocking may be performed by adding and mixing an antibody solution in a colloidal gold solution adjusted to 1 OD/mL, and then adding the component of biological origin to the mixed solution so as to have a final concentration of from 0.1 to 10%. Further, the lower limit of the final concentration is not limited to 0.1 % so that any numerical number such as 0.2, 0.3, 0.4, 0.5, 0.6, 0.7, 0.8, 0.9, or 1% may be selected as the lower limit of the final concentration. In addition, the upper limit of the final concentration is not limited to 10 % so that any numerical number such as 9, 8, 7, 6, 5, 4, 3, or 2% may be selected as the upper limit of the final concentration. In addition, their combination is, for example, any combination of the above-mentioned upper limits and lower limits, such as 0.2 to 9, 0.3 to 8, 0.4 to 7, 0.5 to 6, 0.6 to 5, 0.7 to 4, 0.8 to 3, 0.9 to 2, or 1 to 1.5%. Of those, a final concentration of from 0.5 to 5% may be preferably used.

### (Blocking method)

As a method of blocking the region in the surface of the colloidal gold to which the antibody is not bound with both the components, i.e., the component of non-biological origin and the component of biological origin, not only a method involving separately preparing a mixture of the component of non-biological origin and the component of biological origin, and mixing the mixture and the conjugate to perform the blocking at once, but also a method involving mixing any one of the components and the conjugate, and then mixing the resultant with the other component to perform the blocking in two stages is included in the scope of the present invention.

An example of the former method is performed by, for example, the following steps (1) to (3):
(1) a step of sensitizing the colloidal gold with the antibody to provide a conjugate;
(2) a step of mixing the component of non-biological origin and the component of biological origin to provide a mixture; and
(3) a step of subjecting the surface of the colloidal gold to blocking treatment by mixing the mixture and the conjugate.

In addition, an example of the latter method is performed by, for example, the following steps (A) to (C) or (a) to (c):
(A) sensitizing the colloidal gold with the antibody to provide a conjugate;
(B) subjecting the surface of the colloidal gold to first blocking treatment by mixing the component of non-biological origin and the conjugate; and
(C) subjecting the surface of the colloidal gold to second blocking treatment by adding the component of biological origin to the product of the first blocking treatment in the step (B); or
   (a) sensitizing the colloidal gold with the antibody to provide a conjugate;
   (b) subjecting the surface of the colloidal gold to first blocking treatment by mixing the component of biological origin and the conjugate; and
   (c) subjecting the surface of the colloidal gold to second blocking treatment by adding the component of non-biological origin to the product of the first blocking treatment in the step (b).

### (Detection reagent)

In the present invention, the term "detection reagent" specifically refers to a solution containing at least the conjugate.

The detection reagent may contain, for example, one or more kinds of a stabilizer, a solubilizing aid, and the like for the purpose of maintaining the conjugate in a stable state and promoting a specific reaction of the antibody immobilized on the conjugate with the object to be detected when the conjugate is mixed with the sample, or dissolving or fluidizing the conjugate quickly and effectively. Examples of the stabilizer, the solubilizing aid, and the like may include bovine serum albumin (BSA), sucrose, casein, and amino acids.

In addition, the detection reagent may contain a known sensitizer such as 2-methacryloyloxyethylphosphorylcholine, polyethylene glycol, polyvinylpyrrolidone, dextran, or polyvinyl alcohol as required for the purpose of improving detection sensitivity.

Further, the detection reagent may contain a metal chelating agent for a Ca²⁺ ion or the like, such as EDTA or EGTA as required.

It should be noted that the term "detection" or "measurement" as used herein shouldbe interpreted in the broadest sense, including, for example, the demonstration of the presence of and/or the quantification of the object to be detected, and should not be interpreted in any limiting sense.

### (Antibody to be used in the present invention)

The antibody against a substance to be detected to be used in the present invention has only to be an antibody that specifically reacts with the substance to be detected, and is by no means limited in terms of production method therefor. In addition, the antibody may be a polyclonal antibody or may be a monoclonal antibody. In general, hybridomas that produce the antibody may be produced by subjecting spleen cells of an animal immunized with an antigen as a substance to be detected and myeloma cells of the same species to cell fusion in conformity with Kohler and Milstein's method (see Nature, Volume 256, p 495 (1975)). Alternatively, a monoclonal antibody may be produced by a DNA immunization method, and may be produced with reference to Nature 1992 Mar 12; 356 152-154 or J. Immunol. Methods Mar 1; 249 147-154.

As the antibody to be used in the present invention, instead of the antibody obtained through the step of immunizing an animal or antibody obtained by the DNA immunization method as described above, an antibody obtained by using a genetic recombination technology (e.g., chimeric antibody) may be used, and instead of the whole molecule of an antibody, a functional fragment of an antibody having an antigen-antibody reaction activity may be used. Examples of the functional fragment of an antibody include F(ab')₂ and Fab', and these functional fragments may be manufactured by treating the antibodies obtained as described above with a protease (e.g., pepsin or papain).

In this context, when the antibody to be used is a monoclonal antibody, in the case where the epitope of the antibody for the conjugate (first antibody) is monovalent, a different epitope from that of the first antibody is used as the epitope of the antibody immobilized on the insoluble membrane (second antibody), and in the case where the epitope of the first antibody is multivalent, the second antibody may be an antibody having the same epitope as that of the first antibody, or the first antibody and the second antibody may be the same antibody.

An anti-D-dimer monoclonal antibody is used in Examples to be described later. A preparation method for the anti-D-dimer monoclonal antibody used in the present invention is as described in the next section, but the present invention is not limited thereto, and a commercially available anti-D-dimer monoclonal antibody may be used. Examples of the commercially available anti-D-dimer monoclonal antibody include: clones#DD1 to #DD6 of Hytest Ltd.; and clone#MO1102704 of Fitzgerald Industries International, Inc. In addition, Clone# DD3B6 manufactured by American Diagnostica Inc. may be used. It should be noted that an antibody to be produced by a clone is herein sometimes represented by a clone number or symbol (clone# number or symbol) for convenience.

### (Preparation example of anti-D-dimer monoclonal antibody)

### (1) Preparation of hybridomas

Soluble fibrin produced by subjecting purified human fibrinogen dissolved in PBS to batroxobin treatment was used as an immunogen. The immunogen was mixed and emulsified with complete Freund's adjuvant (manufactured by GIBCO) at 1:1, and 0.1 mg/0.1 mL of the resultant (emulsion) was subcutaneously administered to 6-week-old female BALB/C mice 6 times at intervals of 1 week. Then, 3 days after the final immunization, the spleen was harvested. Spleen cells obtained from the harvested spleen and myeloma cells SP2/O-Agl4 were mixed at a ratio of 6:1, and were subjected to cell fusion in the presence of 50% polyethylene glycol 1540 (manufactured by Wako Pure Chemical Industries, Ltd.). The fused cells were suspended in a HAT medium at 2.5×10⁶/mL as spleen cells, and the suspension was dispensed into the wells of a 96-well culture plate (manufactured by CORNING) at 0.2 mL/well. The plate was incubated in a 5% CO₂ incubator at 37°C, and about 2 weeks later, culture supernatants in wells in which the hybridomas had grown were subjected to the selection of cell strains that produced an antibody reactive to a DD fraction by an ELISA method. Specifically, IgG in each culture supernatant was solid-phased on a microplate (manufactured by NUNC) via a goat anti-mouse IgG (Fc) antibody (manufactured by JACKSON), and then subjected to a reaction with the DD fraction. Further, the resultant was subjected to a reaction with a peroxidase-labeled anti-fibrinogen rabbit polyclonal antibody (manufactured by DAKO). After that, a peroxidase substrate solution containing o-phenylenediamine (manufactured by Tokyo Chemical Industry Co., Ltd.) was added to cause color development, and 1.5 N sulfuric acid was added to stop the color development. After that, measurement was performed with a microplate reader (Abs. 492 nm), and strains that showed high reactivity to the DD fraction were selected. The hybridomas were cloned by a limiting dilution method to establish two kinds of anti-D-dimer monoclonal antibody-producing hybridomas.

### (2) Preparation of monoclonal antibody

To 12-week-old female BALB/C mice that had been intraperitoneally injected with 0.5 mL of pristane 2 weeks earlier, the hybridomas obtained in (1) above were intraperitoneally administered in an amount of 0.5×10⁶ cells. About 2 weeks later, ascitic fluid was collected and centrifuged to provide a supernatant. The supernatant was mixed with an equal amount of a buffer for adsorption (3 mol/L NaCl-1.5 mol/L Glycine-NaOH, pH 8.5), and then mixture was filtered. The filtrate was passed through a protein A column (manufactured by Pharmacia) equilibrated with the buffer for adsorption to adsorb antibodies onto the column, and then the antibodies were eluted from the column with a 0.1 mol/L citrate buffer (pH 3.0) to purify the anti-D-dimer monoclonal antibodies (Clone #672102, and Clone #672101).

### (Sample pad)

In the present invention, a "sample pad" is a part for receiving a measurement sample, and encompasses a pad of any material and shape that, when molded into a pad, can absorb a liquid measurement sample and allow the liquid and a component of the object to be detected to pass through. Specific examples of a material suitable for the sample pad include, but are not limited to, glass fiber, acrylic fiber, a hydrophilic polyethylene material, dry paper, paper pulp, and woven fabric. A pad made of glass fiber is suitably used. The sample pad may also serve as a conjugate pad to be described later. In addition, a generally used blocking reagent may be incorporated into the sample pad as required as long as the incorporation does not deviate from the purposes of the present invention and does not affect the reaction system.

### (Conjugate pad)

In the present invention, the "conjugate pad" is obtained by impregnating a material suitable for the conjugate pad to be described later with the detection reagent that specifically reacts with the substance to be detected, followed by drying. The conjugate pad has a function of allowing the conjugate and the substance to be detected to form a complex when the measurement sample passes through the conjugate pad. The conjugate pad may be disposed alone so as to be brought into contact with the anti-substance-to-be-detected antibody-immobilized membrane. Alternatively, the conjugate pad may be disposed in contact with the sample pad so that the conjugate pad may receive the sample having passed through the sample pad by capillary flow and subsequently transfer the sample by capillary flow to another pad (hereinafter referred to as "3rd Pad") with which the conjugate pad is brought into contact at a different surface from the surface in contact with the sample pad. It should be noted that the selection of parts for one or more kinds of the sample pad and the conjugate pad and how the selected parts are disposed on the antibody-immobilized membrane may be appropriately changed.

The material suitable for the conjugate pad is exemplified by, but not limited to, paper, a cellulose mixture, nitrocellulose, polyester, an acrylonitrile copolymer, glass fiber, or nonwoven fiber such as rayon. A pad made of glass fiber is suitably used.

The conjugate padmay contain, as required, a "control reagent" for securing the reliability of the immunochromatographic method, such as a labeled antibody that does not react with a component of the sample, or a labeled highly antigenic protein such as keyhole limpet hemocyanin (KLH). Those control reagents are components (substances) which have no possibility to exist in the sample, and may be appropriately selected.

### (3rd Pad)

In the present invention, the 3rd Pad may be disposed for the purpose of removing components unnecessary for the detection of the substance to be detected out of the components in the sample and the detection reagent to allow the smooth progress of components necessary for the reaction in the antibody-immobilized membrane. For example, blood cells, insoluble disrupted blood cells, and the like are desirably removed as the components unnecessary for the detection. In addition, the 3rd Pad may also have an additional effect of removing in advance, out of aggregates produced by the antigen-antibody reaction, aggregates that have become too large to move to and smoothly progress in the antibody-immobilized membrane. The 3rd Pad encompasses a pad of any material and shape through which the liquid and a component of the object to be detected can pass. Specific examples thereof include, but not limited to, glass fiber, acrylic fiber, a hydrophilic polyethylene material, dry paper, paper pulp, and woven fabric. A blood cell separation membrane or a similar membrane is suitably used.

### (Immobilization of antibody on insoluble membrane)

The immobilization of the antibody against the substance to be detected in the IC reagent of the present invention on the insoluble membrane may be performed by a generally well-known method. For example, in the case of a flow-through format, the antibody is prepared into a solution having a predetermined concentration, and a certain amount of the solution is applied onto the insoluble membrane in the shape of a particular symbol such as a point or +. In addition, in this case, in order to secure the reliability of the immunochromatographic method, a protein or compound capable of binding to the conjugate is generally immobilized at a position different from that of the antibody against the object to be detected to serve as a "control line". In addition, an antibody against the control reagent may be immobilized at a position different from that of the antibody against the object to be detected to serve as a "control line".

In the case of a lateral-flow format, the immobilization is performed as follows: the antibody is prepared into a solution having a predetermined concentration, and the solution is applied onto the insoluble membrane in a line shape by using, for example, an apparatus having a mechanism capable of horizontally moving a nozzle while discharging the solution from the nozzle at a constant rate. In this case, the concentration of the antibody is preferably 0.1 mg/mL to 5 mg/mL, more suitably 0.5 mg/mL to 2 mg/mL. In addition, the amount of the antibody immobilized on the insoluble membrane may be optimized by adjusting an amount of the solution dropped onto the insoluble membrane in the case of the flow-through format, and may be optimized by adjusting a discharge rate of the solution from the nozzle of the apparatus in the case of the lateral-flow format. In particular, in the case of the lateral-flow format, the amount is suitably 0.5 µL/cm to 2 µL/cm. It should be noted that, in the present invention, the term "flow-through format membrane assay" refers to a format in which a sample liquid or the like progresses so as to perpendicularly pass through the insoluble membrane, and the term "lateral-flow format membrane assay" refers to a format in which the sample liquid or the like progresses so as to move in parallel with the insoluble membrane.

In addition, in the present invention, with regard to the position in the insoluble membrane at which the antibody against the substance to be detected is applied, in the case of the lateral-flow format, the position has only to be set so that the sample liquid may progress from the conjugate pad by capillary action, and sequentially pass through lines in which the antibody against the substance to be detected and a control antibody are respectively applied. The position is preferably set so that the line in which the antibody against the substance to be detected is applied may be positioned upstream, and the line in which the control antibody is applied may be positioned downstream thereof. In this case, a distance between the respective lines is desirably set to be sufficient to allow the detection of a signal from the label. Also in the case of the flow-through format, the position at which the antibody against the substance to be detected is applied has only to be set so as to allow the detection of the signal from the label.

The antibody solution to be applied onto the insoluble membrane may be generally prepared by using a predetermined buffer. The kind of the buffer may be, for example, a generally used buffer such as a phosphate buffer, a Tris buffer, or Good's buffer. The pH of the buffer, which preferably falls within the range of 6.0 to 9.5, has only to be appropriately set depending on the properties of the antibody to be used. For example, a buffer having a pH of 9 . 0 maybe used for an anti-D-dimermonoclonal antibody to be described later. The buffer may further contain, for example, salts such as NaCl, a stabilizer or preservative such as sucrose, or an antiseptic such as ProClin. The salts include one to be added for adjusting ionic strength, such as NaCl, and one to be added in the step of adjusting the pH of the buffer, such as sodium hydroxide. After the immobilization of the antibody on the insoluble membrane, blocking may be further performed by bringing a generally used blocking agent into a solution or vapor state and coating portions except for the antibody-immobilized portion with the blocking agent. The insoluble membrane having immobilized thereon the antibody as described above is herein sometimes referred to as "antibody-immobilized membrane".

### (Insoluble membrane)

In the present invention, as the insoluble membrane (hereinafter sometimes simply referred to as membrane), one made of any material may be used. Examples thereof include, but not limited to, polyethylene, polyethylene terephthalate, nylons, glass fibers, polysaccharides such as cellulose and cellulose derivatives, and ceramics. Specific examples thereof may include glass fiber filter paper, cellulose filter paper, and the like sold by Millipore, Toyo Roshi Kaisha, Ltd., Whatman, and the like. In addition, by appropriately selecting the pore diameter and structure of the insoluble membrane, it is possible to control the rate at which an immune complex of the colloidal gold-labeled antibody and the substance to be detected flows in the membrane. Through the control of the flow rate in the membrane, the amount of the labeled antibody to be bound to the antibody immobilized on the membrane can be adjusted. Accordingly, the pore diameter and structure of the membrane are desirably optimized in consideration of its combination with other constituent materials for the test strip for immunochromatography of the present invention.

### (Absorbent pad)

In the present invention, an absorbent pad is a part having liquid absorbability to control the progress of the measurement sample by absorbing the measurement sample that has moved in or passed through the insoluble membrane. The absorbent pad has only to be provided in the most downstream part of the strip construction in the lateral-flow format, and has only to be provided, for example, downstream of the antibody-immobilized membrane in the flow-through format. For example, filter paper may be used as the absorbent pad, but the absorbent pad is not limited thereto. 740-E of Whatman or the like is suitably used.

### (Test strip for immunochromatography)

The test strip for immunochromatography of the present invention includes at least the following components:
(1) a conjugate pad that carries a conjugate including colloidal gold having a surface sensitized with a first antibody, the surface of the colloidal gold being blocked with both components, i.e., a component of non-biological origin and a component of biological origin; and
(2) an insoluble membrane that includes a second antibody that binds to a complex of the conjugate and a substance to be detected, the insoluble membrane being disposed downstream of the conjugate pad. The test strip for immunochromatography encompasses, in addition to the above-mentioned construction, one on which any one or more of the sample pad, the absorbent pad, and the 3rd pad are further disposed and mounted. The components of the test strip are generally arranged on a solid phase support such as a plastic pressure-sensitive adhesive sheet. It is apparent that not only the solid phase support is constructed with a substance that does not hinder the capillary flow of the measurement sample, but also a substance that does not hinder the capillary flow of the measurement sample is used as an adhesive component. It should be noted that a polyester film or the like may be used for lamination for the purposes of enhancing the mechanical strength of the antibody-immobilized membrane and preventing the evaporation (drying) of water during the assay.

### (Detection device)

The test strip for immunochromatography of the present invention may be used by being housed/mounted in an appropriate container (housing) designed in consideration of, for example, the size of the strip, a method of adding the measurement sample and the position at which the measurement sample is added, the position in the antibody-immobilized membrane at which the antibody is immobilized, and a detection method for the signal, and the test strip in such housed/mounted state is referred to as "device".

### (Others)

Herein, the "insoluble membrane" is sometimes referred to as "solid phase", and the act of supporting the antigen or the antibody on the insoluble membrane physically or chemically or the state of the antigen or antibody so supported is sometimes described with the term "fix", "immobilize", "solid-phase", "sensitize", "adsorb", or "carry".

### (Sample)

In a detection method of the present invention, a "sample" containing a substance to be detected may be exemplified by, but not limited to, blood, serum, plasma, urine, saliva, phlegm, pancreas extract, lacrimal fluid, otorrhea, and prostatic fluid. In general, whole blood, serum, or plasma is preferred. The term "specimen" is herein used in the same meaning as "sample".

### (Object to be detected)

The obj ect to be detected of the present invention is a substance present in blood, serum, plasma, urine, saliva, phlegm, pancreas extract, lacrimal fluid, otorrhea, prostatic fluid, or the like. Examples thereof include: inflammation-related markers such as C-reactive protein (CRP), IgA, IgG, and IgM; coagulation and fibrinolysis markers such as a fibrin degradation product (e.g., a D-dimer), soluble fibrin, thrombin-antithrombin complex (TAT), plasmin-plasmin inhibitor complex (PIC), and plasminogen (PLG); cardiovascular-related markers such as oxidized LDL, brain natriuretic peptide (BNP), and lipoprotein(a) (Lp(a)); metabolism-related markers such as adiponectin and hemoglobin A1c (HbA1c); tumor markers such as carcinoembryonic antigen (CEA), α-fetoprotein (AFP), CA19-9, CA125, and prostate-specific antigen (PSA); infectious disease-related markers such as a hepatitis B virus (HBV), a hepatitis C virus (HCV), *Chlamydia trachomatis,* and *Neisseria gonorrhoeae;* allergen-specific immunoglobulin E (IgE); hormones; and drugs. Of those, multivalent antigens such as a D-dimer, CRP, Lp(a), and PLG, and trace components such as BNP are more preferred.

### (Diluting liquid)

In the present invention, when the sample needs to be diluted depending on the concentration of the object to be detected in the sample, a diluting liquid may be used. As the diluting liquid, a diluting liquid having any composition may be used as long as the diluting liquid has an action of not making it impossible to detect a signal of the antigen-antibody reaction depending on the antigen concentration by, for example, markedly inhibiting the antigen-antibody reaction or, conversely, markedly promoting the reaction to cause a failure in progress by capillary action because of excessive aggregation of the label. Examples of the diluting liquid having such action include purified water, physiological saline, and low-concentration buffers each having a pH of 6.0 to 10. 0, such as a 10 mmol/L to 20 mmol/L phosphate buffer, a 10 mmol/L to 20 mmol/L Tris-HCl buffer, and a 10 mmol/L to 20 mmol/L glycine-HCl buffer. In addition, for the purpose of controlling the progress rate of the sample liquid in the strip, a surfactant may be added to any such diluting liquid.

### (Measurement)

As a method of quantifying a signal derived from the colloidal gold, it suffices to measure absorbance or reflected light intensity in accordance with a known method. Through extrapolation from the amounts of changes in the absorbance or the reflected light intensity to a calibration curve of samples having known concentrations, the concentration of the substance to be detected can also be measured.

### Examples

Next, the present invention is specifically described by way of Examples, but the scope of the present invention is not limited thereto.

### (Test Example 1) Study on blocking agent 1

Colloidal gold after antibody sensitization was subjected to blocking treatment for the surface of the colloidal gold with a commercially available blocking agent of biological origin or non-biological origin.

### (1) Test materials

### (i) Colloidal gold solution

To 500 mL of purified water heated to 65°C was added 0.7 mL of an aqueous solution having dissolved therein triammonium citrate and trisodium citrate dihydrate each at a ratio of 7% (w/v), and the whole was mixed by stirring. Subsequently, 1 mL of a 7% (w/v) aqueous solution of tetrachloroauric (III) acid was added, the mixture was subj ected to a reaction for 10 minutes while being stirred, and then the reaction solution was boiled. After that, the resultant was cooled in ice water to prepare a colloidal gold solution having an average particle diameter of 30 nm. The colloidal gold solution having an average particle diameter of 30 nm was adjusted to 10D/mL with a 20 mmol/L Tris buffer (pH 7.5).

### (ii) Blocking agent to be tested

### Component of non-biological origin

·N101 (manufactured by NOF CORPORATION)
·N102 (manufactured by NOF CORPORATION)
·SN100 (manufactured by NOF CORPORATION)
·NB4025 (manufactured by COSMO BIO CO., LTD.)
·Stabil Guard (manufactured by SurModics Inc.)
·Protein-Free Blocking Buffer (Thermo Fisher Scientific Inc.) Component of biological origin
·BSA (manufactured by Desert Biologicals)
·Blocking Peptide Fragment (manufactured by TOYOBO CO., LTD.)
·Neo Protein Saver (manufactured by TOYOBO CO., LTD.)
·StartingBlock™ (PBS) Blocking Buffer (manufactured by PIERCE)
·Casein (manufactured by VWR International, LLC.)

### (2) Test method

To 20 mL of the 1 OD/mL colloidal gold solution (pH 7.5) was added 1 mL of an anti-D-dimer monoclonal antibody (Clone#672102) prepared as a solution in a 10 mmol/L Tris-HCl buffer (pH 7.5), and the mixture was stirred at room temperature for 10 minutes. In the case of the blocking agent of "non-biological origin", its undiluted solution in the following amount was added to the mixed solution of the colloidal gold and the antibody. In addition, in the case of the blocking agent of "biological origin", the blocking agent was dissolved in a 20 mmol/L Tris buffer to be prepared as a 10% (w/v) solution, and then 2 mL thereof were added to the mixed solution of the colloidal gold and the antibody. The mixture was stirred for 5 minutes, and then centrifuged at 10°C and 11,900xg for 45 minutes.

The supernatant was removed, and then to the resultant residue were added 800 µL of a 20 mmol/L Tris buffer (pH 7.2) to suspend the conjugate of each case. The suspension was measured for its absorbance at the maximum absorption wavelength (λmax) and absorbance at a wavelength of 600 nm, and their ratio (A₆₀₀nm/Aλₘₐₓ) was calculated.

### <Addition amount of blocking agent of non-biological origin>

N101, N102, Stabil Guard, Protein-Free Blocking Buffer 2 mL
SN100, NB4025, Protein-Free Blocking Buffer 0.2 mL

### (3) Test results and discussion

Table 1 shows the test results. As apparent from the results, when the antibody-sensitized colloidal gold was subjected to blocking treatment with N101 or N102, then centrifuged to collect the antibody-sensitized colloidal gold, and an attempt was made to resuspend the colloidal gold in a 20 mmol/L Tris buffer (pH 7.2), the colloidal gold aggregated and was unable to be resuspended, with the result that absorbance measurement was unable to be performed. In addition, in the case of treatment with NB4025, SN100, Stabil Guard, or Protein-Free, the resuspension after centrifugation was similarly poor, though to a lesser degree than those of the above-mentioned two kinds, and the absorbance showed a high value as well. On the other hand, in each of the case of treatment with the component of biological origin, i.e., BSA, Blocking Peptide Fragment, Neo Protein Saver, Starting Block, or casein, the colloidal gold did not aggregate and was able to be resuspended, and the absorbance showed a low value as well.

As seen from the above, the colloidal gold subj ected to blocking treatment with the component of biological origin was excellent in dispersibility when resuspended after the centrifugation of the antibody-sensitized colloidal gold and was usable as a conjugate. On the other hand, the colloidal gold subjected to blocking treatment with the component of non-biological origin had a problem in that the antibody-sensitized colloidal gold aggregated by centrifugation, and consequently, was unable to be resuspended or markedly lacked stability.

**[Table 1]**

| Blocking agent | Origin | State of colloidal gold when subjected to centrifugation → resuspension | Absorbance ratio (A₆₀₀ₙₘ/A_{λmax}) |
|---|---|---|---|
| BSA | Biological | Able to be resuspended | 0.169 |
| N101 | Non-biological | Aggregated →unable to be resuspended | - |
| N102 | Non-biological | Aggregated →unable to be resuspended | - |
| NB4025 | Non-biological | Somewhat aggregated → poor in stability | 0.252 |
| SN100 | Non-biological | Somewhat aggregated → poor in stability | 0.248 |
| Stabil Guard | Non-biological | Somewhat aggregated → poor in stability | 0.212 |
| Protein-Free | Non-biological | Somewhat aggregated → poor in stability | 0.224 |
| Blocking Peptide Fragment | Biological | Satisfactorily resuspended | 0.148 |
| Neo Protein Saver | Biological | Satisfactorily resuspended | 0.148 |
| Starting Block | Biological | Satisfactorily resuspended | 0.166 |
| Stabil Coat | Biological | Satisfactorily resuspended | 0.167 |
| Casein | Biological | Satisfactorily resuspended | 0.145 |

### (Test Example 2) Study on blocking agent 2

After the blocking treatment with the component of non-biological origin of Test Example 1, additional blocking treatment with the component of biological origin was performed to study the dispersibility of the treated colloidal gold.

### (1) Test method

To 20 mL of the 1 OD/mL colloidal gold solution (pH 7.5) was added 1 mL of an anti-D-dimer monoclonal antibody (Clone#672102) prepared as a solution in a 10 mM Tris-HCl buffer (pH 7.5), and the mixture was stirred at room temperature for 10 minutes. To the mixed solution of the colloidal gold and the antibody was added one of the components of non-biological origin, i.e., 1 mL of N101 (Formulation 2), 1 mL of N102 (Formulation 3), 0.2 mL of NB4025 (Formulation 4), 0.2 mL of SN100 (Formulation 5), 2 mL of Stabil Guard (Formulation 6), or 2 mL of Protein-Free Blocking Buffer (Formulation 7), and then the mixture was stirred at room temperature for 5 minutes. Further, the blocking agent of biological origin, i.e. , 1 mL of BSA (10% (w/v), 20 mM Tris buffer (pH 7.5) was added thereto, and the mixture was stirred at room temperature for 5 minutes. After that, the mixture was centrifuged at 10°C and 11,900xg for 45 minutes. The supernatant was removed, and then to the resultant residue were added 800 µL of a 20 mM Tris buffer (pH 7.2) to suspend the conjugate of each case. The suspension was measured for its absorbance at the maximum absorption wavelength and absorbance at a wavelength of 600 nm. In addition, in accordance with the method of Test Example 1, a conjugate blocked with only BSA was prepared (Formulation 1), and used as a control.

### (2) Test results and discussion

Table 2 shows the results. In Test Example 1, as shown in Table 1, the blocking treatment with the component of non-biological origin alone resulted in a poor state of resuspension after the centrifugation of the colloidal gold. However, through the addition of the blocking treatment with the component of biological origin to the blocking treatment with the component of non-biological origin as in this test, dispersibility of the colloidal gold at the time of resuspension became satisfactory, and the antibody-sensitized colloidal gold was able to be collected. In addition, the absorbance showed a measurement value as low as that in the case of blocking with BSA alone as well.

Therefore, blocking treatment with the component of non-biological origin, which had heretofore not been usable as a blocking agent for antibody-sensitized colloidal gold, became possible, and it was demonstrated that it was possible to control measurement sensitivity depending on the kind of blocking agent.

**[Table 2]**

| Formulation | Blocking agent | State of colloidal gold at time of centrifugation → resuspension | Absorbance ratio (A₆₀₀ₙₘ/A_{λmax}) |
|---|---|---|---|
| Formulation 1 | BSA | Satisfactorily resuspended | 0.169 |
| Formulation 2 | N101+BSA | Satisfactorily resuspended | 0.141 |
| Formulation 3 | N102+BSA | Satisfactorily resuspended | 0.159 |
| Formulation 4 | NB4025+BSA | Satisfactorily resuspended | 0.169 |
| Formulation 5 | SN100+BSA | Satisfactorily resuspended | 0.173 |
| Formulation 6 | Stabil Guard+BSA | Satisfactorily resuspended | 0.174 |
| Formulation 7 | Protein-Free+BSA | Satisfactorily resuspended | 0.151 |

### (Example 1) Measurement using conjugate of the present invention 1

A study was made on sensitivity and a measurement range in the case where the surface of colloidal gold was subj ected to blocking treatment with both the components, i.e., the component of biological origin and the component of non-biological origin. 1. Production example of IC device of the present invention (1) Colloidal gold-labeled anti-D-dimer antibody (conjugate)

The anti-D-dimer antibody-sensitized conjugates of Formulation 1 to Formulation 7 obtained in Test Example 2 were used.

### (2) Production of conjugate pad

The anti-D-dimer monoclonal antibody-sensitized conjugates prepared in (1) above were each mixed at 4.1 OD/mL with a 20 mmol/L Tris buffer (pH 7.2) containing a 2.4% lactose solution to produce a conjugate solution. A pad made of glass fiber (Pall Corporation, No. 8964) having a certain volume was soaked with a 1.2-fold volume of the conjugate solution with respect to the volume of the pad. The resultant was dried by being heated in a dry oven at 70°C for 40 minutes to provide a conjugate pad. In addition, when an additive such as a sensitizer is added as required, the same operation has only to be performed after the addition of a required amount of the additive to the conjugate solution.

### (3) Production of anti-D-dimer antibody-immobilized membrane

An anti-D-dimer monoclonal antibody (Clone# DD3B6, manufactured by Sekisui Diagnostics) was prepared as a 1.5 mg/mL solution in a 10 mmol/L Tris buffer (pH 9.0) containing 2.5% sucrose. In addition, for the purpose of color development of a control line, a fibrin degradation product was diluted with a 10 mmol/L Tris-HCl buffer (pH 7.2) containing 2.5% sucrose to prepare a 3 mg/mL solution. The solutions were applied in line shapes onto a nitrocellulose membrane (Millipore, HF180) so that the anti-D-dimer monoclonal antibody was positioned inside one end of a short side of the membrane and the fibrin degradation product was positioned at an interval of about 5 mm therefrom using a dispenser for IC "XYZ3050" (BIO DOT) set at 0.8 µL/cm. The resultant was dried in a dry oven at 40°C for 45 minutes to provide an antibody-immobilized membrane. It should be noted that the "fibrin degradation product" is prepared by hydrolysis of stable fibrin with plasmin, and undergoes color development through the binding of a conjugate sensitized with an anti-D-dimer monoclonal antibody to the fibrin degradation product to provide a control line for assuring the performance of the IC reagent.

### (4) Production of sample pad

A pad made of glass fiber (Lydall) was appropriately cut to a needed size and used.

### (5) Production of test strip for immunochromatography

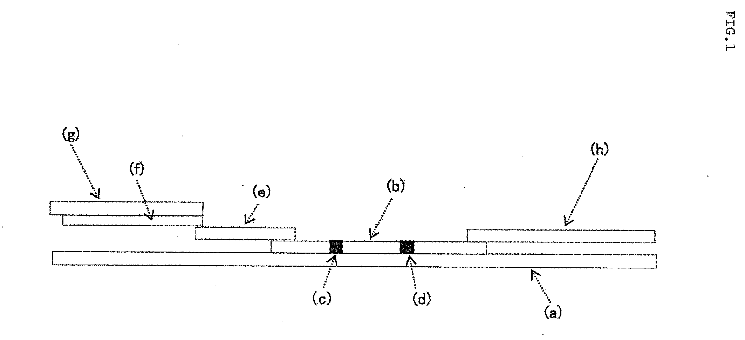

An antibody-immobilized membrane (b) described above was attached to a plastic pressure-sensitive adhesive sheet (a), application sites were set in the order of an anti-D-dimer antibody (c) and a fibrin degradation product (d) from an upstream side in the direction of progress, and a 3rd Pad (e) was further mounted. Next, a conjugate pad (f) produced in (2) above was disposed and mounted, a sample pad (g) produced in (4) above was further disposed and mounted so as to overlap the conjugate pad, and an absorbent pad (h) was disposed and mounted at the opposite side end. The structure in which the respective constituent elements were thus stacked together was cut to produce a test strip for immunochromatography. In an assay, the test strip was used in the form of an IC test device by being housed/mounted in a dedicated plastic housing (having a sample addition window and a detection window, not shown in FIG. 1). FIG. 1 illustrates a schematic construction view of the test strip for immunochromatography.

### (6) Study on measurement sensitivity and measurement range

Respective test strips for immunochromatography using the conjugates of the seven formulations described above were used to study the measurement range of a D-dimer. As specimens, there were prepared specimens at various concentration levels, i.e., 0.6, 1.5, 5.3, 10.7, and 21.8 µg/mL by adding a fibrin degradation product to commercially available human citrated plasma. 120 µL each of the specimens were added through the sample addition window of the test device, and the reflected light intensity of the detection window of the test device 10 minutes after the addition was measured with an IC reader ICA-1000 (Hamamatsu Photonics K.K.).

### (7) Test results and discussion

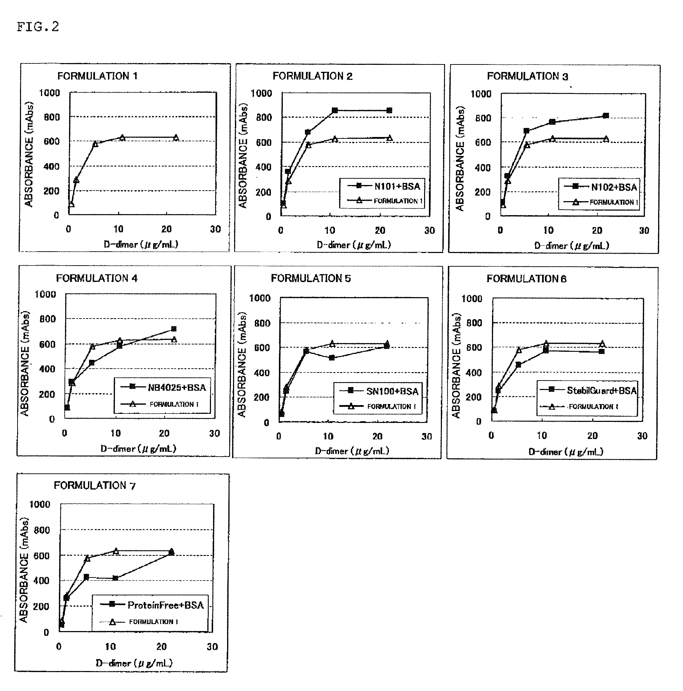

FIGS. 2 show the results. Formulation 2 to Formulation 7, in which blocking was performed with BSA and the component of non-biological origin, each showed sensitivity equivalent to or higher than that of Formulation 1, in which blocking was performed with only BSA.

In particular, Formulation 2 and Formulation 3 showed remarkably high sensitivity as compared to Formulation 1. In addition, in Formulation 4, the absorbance was observed to increase to higher D-dimer concentrations as compared to Formulation 1, and an evidently broad measurement range was able to be obtained.

Specifically, Formulations 6 and 7 showed almost equivalent results to that of Formulation 1 in a high concentration region (20 µg/mL), but their amounts of change in absorbance (Δabsorbance) in a low concentration region were smaller. The results suggest that, through the optimization of conditions, the measurement sensitivity can be adjusted to lower values as well in each of the formulations.

The fact that Formulation 2 to Formulation 7 provide sensitivity and a measurement range different from each other is probably due to a difference among the respective components of non-biological origin adsorbed onto the surface of the colloidal gold after antibody sensitization.

The component of non-biological origin causes aggregation of conjugates (Test Example 1), and hence has hitherto been considered unsuitable for use as a blocking agent. However, the inventors of the present invention have newly found the actions of the component of non-biological origin to adjust sensitivity and expand a measurement range, and demonstrated that the component of non-biological origin can be suitably used in the immunoassaymethod.

### (Example 2) Measurement using conjugate of the present invention 2

A study was made on sensitivity and a measurement range in the case where the surface of colloidal gold was blocked with both the components, i.e., the component of biological origin and the component of non-biological origin.

### 1. Production of IC device of the present invention

### (1) Test method

### (i) Preparation of anti-D-dimer antibody-sensitized conjugate

To 20 mL of the 1 OD/mL colloidal gold solution (pH 7.5) was added 1 mL of an anti-D-dimer monoclonal antibody (Clone#672102) prepared as an 80 µg/mL solution in a 10 mM Tris-HCl buffer (pH 7.5), and the mixture was stirred at room temperature for 10 minutes. 1 mL of the blocking agent as the component of non-biological origin was added to the mixed solution of the colloidal gold and the antibody, and then the mixture was stirred at room temperature for 5 minutes. Further, 1 mL or 2 mL of the blocking agent of biological origin were added thereto, and then the mixture was stirred at room temperature for 5 minutes. Table 3 shows the combination of the blocking agents added in the above procedure and their addition amounts. After the stirring, the resultant was centrifuged at 10°C and 11, 900xg for 45 minutes. The supernatant was removed, and then to the resultant residue were added 800 µL of a 20 mM Tris buffer (pH 7.2) to suspend the conjugate of each formulation. Thus, an anti-D-dimer antibody-sensitized conjugate was obtained.

**[Table 3]**

| Formulation | Blocking agent of non-biological origin | Addition amount | Blocking agent of biological origin | Addition amount |
|---|---|---|---|---|
| Formulation 8 | - | - | 10% (w/v) BPF, 10 mM Tris buffer (pH7.5) | 1 mL |
| Formulation 9 | N102 | 1 mL | 10% (w/v) BPF, 10 mM Tris buffer (pH 7.5) | 1 mL |
| Formulation 10 | N102 | 1 mL | 10% (w/v) BSA, 10 mM Tris buffer (pH 7.5) | 1 mL |
| Formulation 11 | N102 | 1 mL | 10% (w/v) Neo Protein Saver, 10 mM Tris buffer (pH 7.5) | 1 mL |
| Formulation 12 | N102 | 1 mL | Starting Block™ (PBS) Blocking Buffer | 1 mL |
| Formulation 13 | N102 | 1 mL | Stabil Coat | 1 mL |
| Formulation 14 | N102 | 1 mL | 1.5% (w/v) Casein, 10 mM Tris buffer (pH 7.5) | 2 mL |
| Formulation 15 | N101 | 1 mL | 10% (w/v) BPF, 10 mM Tris buffer (pH 7.5) | 1 mL |
| Formulation 16 | NB4025 | 1 mL | 10% (w/v) BPF, 10 mM Tris buffer (pH 7.5) | 1 mL |
| Formulation 17 | SN100 | 1 mL | 10% (w/v) BPF, 10 mM Tris buffer (pH 7.5) | 1 mL |
| Formulation 18 | Stabil Guard | 1 mL | 10% (w/v) BPF, 10 mM Tris buffer (pH 7.5) | 1 mL |
| Formulation 19 | Protein-Free Blocking Buffer | 1 mL | 10% (w/v) BPF, 10 mM Tris buffer (pH 7.5) | 1 mL |

### (ii) Production of IC device

An IC device was produced by the same method as that described in Example 1 with each of the anti-D-dimer antibody-sensitized conjugates produced in (i).

### (iii) Study on measurement sensitivity and measurement range

The respective IC devices of the twelve formulations of (ii) were used to study the measurement sensitivity and measurement range of a D-dimer. As specimens, there were prepared specimens at various concentration levels, i.e., 0.75, 1.4, 5.0, 8.5, and 16.0 µg/mL by adding a fibrin degradation product to commercially available human citrated plasma. 120 µL each of the specimens were added through the sample addition window of each test device, and the reflected light intensity of the detection window of the test device 10 minutes after the addition was measured with an IC reader ICA-1000 (Hamamatsu Photonics K.K.).

### (2) Test results and discussion

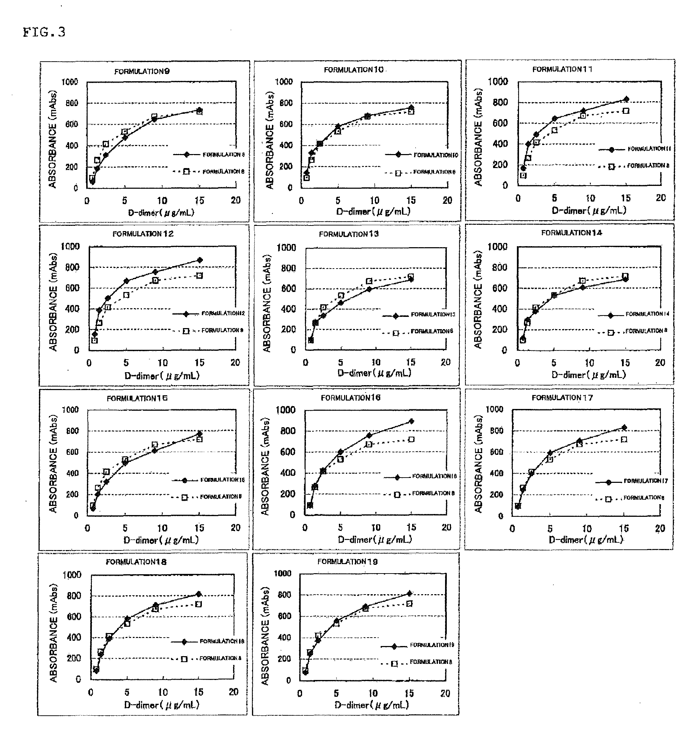

FIGS. 3 show the results. Formulation 9 to Formulation 14, in which blocking was performed with N102 and the respective components of biological origin, each showed sensitivity equivalent to or higher than that of Formulation 8, in which blocking was performed with only BPF. In addition, Formulations 15 to 19, in which blocking was performed with the respective components of non-biological origin and BPF, also each showed sensitivity equivalent to or higher than that of Formulation 8, in which blocking was performed with only BPF.

In particular, Formulation 12 and Formulation 16 showed remarkably high sensitivity as compared to Formulation 8. In addition, as compared to Formulation 8, in Formulations 11 and 12, and Formulations 15 to 19, the amounts of change in absorbance (Δabsorbance) were observed to increase even in high concentration regions, and evidently broad measurement ranges were able to be obtained.

Specifically, Formulations 11 and 12 showed higher measurement values in all concentration regions than those of Formulation 8. The results suggest that, through the optimization of conditions, the measurement sensitivity can be adjusted to higher values as well.

The fact that Formulation 9 to Formulation 19 provide sensitivity and a measurement range different from each other is probably due to a difference among the respective components of non-biological origin adsorbed onto the surface of the colloidal gold after antibody sensitization.

### (Example 3) Blocking order

A study was made on the influence of the addition order of the blocking agent based on the component of non-biological origin and the blocking agent based on the component of biological origin.

### ·Test method

IC devices of three formulations were produced in which combinations of Table 4 were adopted as the addition order of the respective blocking agents in the preparation of an anti-D-dimer antibody-sensitized conjugate. The test was performed in accordance with the same method as that of Example 2 except for the blocking method. After that, measurement sensitivity and a measurement range were studied in the same manner as in Example 2.

**[Table 4]**

| | Addition order 1 | Addition amount | Addition order 2 | Addition amount |
|---|---|---|---|---|
| Formulation 9 | N102 | 1 mL | 10% (w/v) BPF, 10 mM Tris buffer (pH7.5) | 1 mL |
| Formulation 21 | 10% (w/v) BPF, 10 mM Tris buffer (pH 7.5) | 1 mL | N102 | 1 mL |
| Formulation 22 | Solution obtained by mixing equal amounts of N102 and 10% (w/v) BPF, 10 mM Tris buffer (pH 7.5) | 2 mL | - | - |

### (2) Test results and discussion

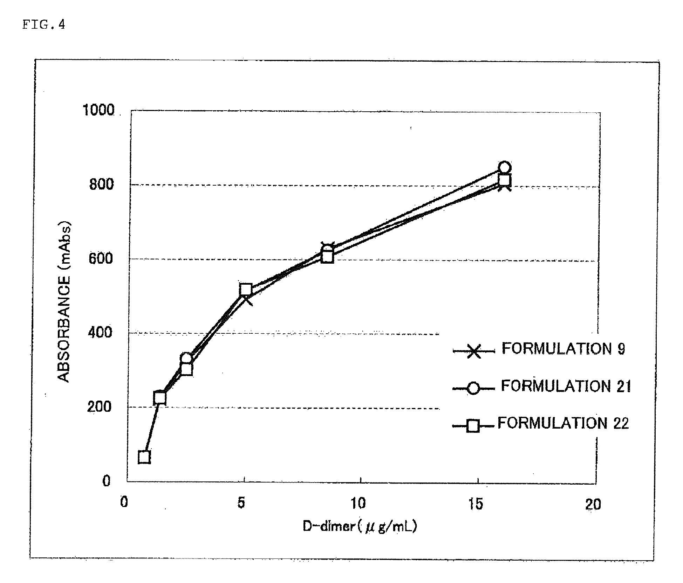

FIG. 4 shows the results. Each of the formulations showed equivalent sensitivity. The effects of the present invention are obtained irrespective of the blocking order. A person skilled in the art can select an appropriate addition order depending on the combination with other constituent elements to be used in the assay.

### (Example 4) Usable concentration range

A study was made on a usable concentration range for each blocking agent.

### (1) Test method

Measurement was performed by the same method as that of Example 2 except that combinations of the addition amounts of the respective blocking agents in the preparation of the anti-D-dimer antibody-sensitized conjugate (concentrations after addition) were in accordance with Table 5.

**[Table 5]**

| | 30-nm colloidal gold at 1 OD | Amount of added antibody solution | Blocking agent of non-biological origin | | | Blocking agent of biological origin | | |
|---|---|---|---|---|---|---|---|---|
| | | | Blocking agent | Addition amount | Final concentration | Blocking agent | Addition amount | Final concentration |
| Formulation 23 | 20 mL | 1 mL | 2.2% N102 | 1 mL | 0.1% | 2% BPF, 10 mM Tris buffer at pH 7.5 | 1. 15 mL | 0.10% |
| Formulation 24 | 20 mL | 1 mL | 2.2% N102 | 1 mL | 0.1% | 20% BPF, 10 mM Tris buffer at pH 7.5 | 11 mL | 10.0% |
| Formulation 25 | 19 mL | 1 mL | N102 | 3.55 mL | 15.1% | 2% BPF, 10 mM Tris buffer at pH 7.5 | 1.2 mL | 0.10% |
| Formulation 26 | 20 mL | 1 mL | N102 | 3.7 mL | 15.0% | 20% BPF, 10 mM Tris buffer at pH 7.5 | 24.7 mL | 10.0% |

### (2) Test results and discussion

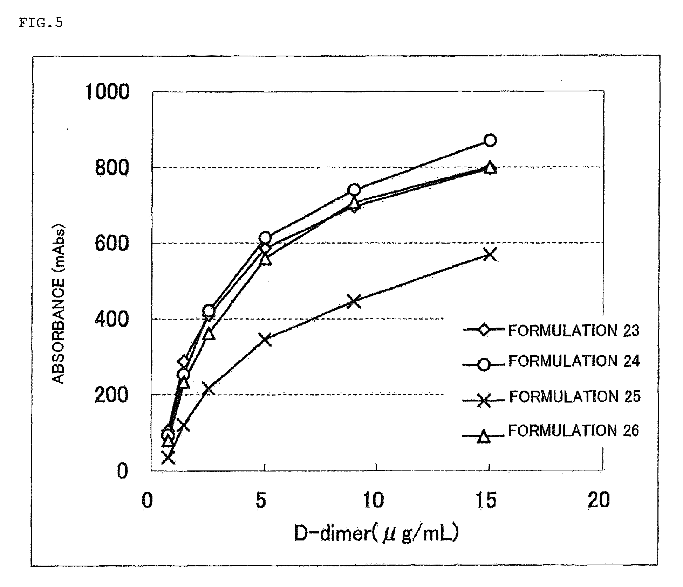

As shown in FIG. 5, it was found that the respective blocking agents were usable in the following ranges: component of non-biological origin: 0.1 to 15.0%; and component of biological origin: 0.1 to 10%.

In addition, with attention to the ratio of the component of non-biological origin to the component of biological origin, it is found that sensitivity can be decreased by increasing the ratio. Specifically, the ratio is lowest in Formulation 24 (1/10), and increases in the order of Formulation 23 (1-fold), Formulation 26 (1.5-fold), and Formulation 25 (150-fold). In addition, the sensitivity was found to decrease in this order. Thus, from the above-mentioned results and Example 1, it is found that the measurement sensitivity can be adjusted through the selection of the kind of component of non-biological origin, and the selection of the ratio of the component of non-biological origin to the component of biological origin.

### (Example 5) Correlation with control method

A latex agglutination turbidimetric immunoassay method (LTIA method), which had already been established as a measurement method for a D-dimer, was employed as a control method to study a correlation.

### (1) Test method

The IC devices of Formulation 8 and Formulation 9 in Example 2 were used, and 29 cases of citrated plasma of human origin were subjected to measurement by the same method as that of Example 2. In the control method, Nanopia D-dimer (manufactured by SEKISUI MEDICAL CO., LTD.) was used, and the concentration of the D-dimer in the citrated plasma of each case was measured with a model 7170 automatic analyzer (manufactured by Hitachi High-Technologies Corporation). For a relationship of the measurement values in each of Formulation 8 and Formulation 9 with the measurement values in the control method, statistical processing was performed by Spearman's rank sum test to determine a correlation coefficient. That is, the measurement values of each of the control method, Formulation 8, and Formulation 9 were ranked in ascending order, and a correlation coefficient between the ranks of the control method and the ranks of each formulation was calculated.

### (2) Test results and discussion

Table 6 shows the results. With regard to the correlation coefficient between behaviors (ranks) of the measurement values of the respective formulations and the control method, the IC device of Formulation 9 showed a more satisfactory correlation as compared to the IC device of Formulation 8. That is, it was demonstrated that the use of the IC device produced by the method of the present invention enabled not only the adj ustment of measurement sensitivity and the expansion of a measurement range, but also more accurate measurement.

**[Table 6]**

| | Correlation coefficient |
|---|---|
| Formulation 8 | 0.9685 |
| Formulation 9 | 0.9813 |

### Reference Signs List

(a) plastic pressure-sensitive adhesive sheet
(b) antibody-immobilized membrane
(c) anti-D-dimer antibody
(d) fibrin degradation product
(e) 3rd Pad
(f) conjugate pad
(g) sample pad
(h) absorbent pad

## Claims

1. A conjugate, comprising a colloidal metal having a surface sensitized with an antibody or an antigen, wherein the surface of the colloidal metal is blocked with a component of non-biological origin and a component of biological origin.

2. The conjugate according to claim 1, wherein the component of biological origin comprises one or more members selected from the group consisting of: BSA; casein; sericin; a polypeptide corresponding to amino acids 419 to 607 of an amino acid sequence of DnaK, which is a heat shock protein (HSP) of *Escherichia coli* origin; NEO PROTEIN SAVER; StartingBlock™ (PBS) Blocking Buffer; and Stabil Coat.

3. The conjugate according to claim 1 or 2, wherein the component of non-biological origin comprises one or more members selected from the group consisting of: a methacrylic acid polyoxyalkylene ester; NB4025; SN100; Stabil Guard; and Protein-Free Blocking Buffer.

4. The conjugate according to any one of claims 1 to 3, wherein the methacrylic acid polyoxyalkylene ester comprises one or more members selected from the group consisting of: polyoxyethylene monomethacrylate; polyoxyethylene polyoxytetramethylene monomethacrylate; polyoxyethylene polyoxypropylene monomethacrylate; and octylpolyoxyethylene polyoxypropylene monomethacrylate.

5. The conjugate according to any one of claims 1 to 4, wherein the colloidal metal is colloidal platinum or colloidal gold.

6. A detection method for a substance to be detected in a sample, the detection method comprising bringing the sample into contact with a conjugate comprising a colloidal metal having a surface sensitized with an antibody or an antigen,
the surface of the colloidal metal being blocked with a component of non-biological origin and a component of biological origin.

7. The detection method according to claim 6, wherein the conjugate is carried on a test strip for immunochromatography.

8. The detection method according to claim 6 or 7, wherein the component of biological origin comprises one or more members selected from the group consisting of: BSA; casein; sericin; a polypeptide corresponding to amino acids 419 to 607 of an amino acid sequence of DnaK, which is a heat shock protein (HSP) of *Escherichia coli* origin; NEO PROTEIN SAVER; StartingBlock™ (PBS) Blocking Buffer; and Stabil Coat.

9. The detection method according to any one of claims 6 to 8, wherein the component of non-biological origin comprises one or more members selected from the group consisting of: a methacrylic acid polyoxyalkylene ester; NB4025; SN100; Stabil Guard; and Protein-Free Blocking Buffer.

10. The detection method according to any one of claims 6 to 9, wherein the methacrylic acid polyoxyalkylene ester comprises one or more members selected from the group consisting of: polyoxyethylene monomethacrylate; polyoxyethylene polyoxytetramethylene monomethacrylate; polyoxyethylene polyoxypropylene monomethacrylate; and octylpolyoxyethylene polyoxypropylene monomethacrylate.

11. The detection method according to any one of claims 6 to 10, wherein the substance to be detected comprises amultivalent antigen.

12. The detection method according to claim 11, wherein the substance to be detected comprises a D-dimer.

13. The detection method according to any one of claims 6 to 12, wherein the colloidal metal is colloidal platinum or colloidal gold.

14. A test strip for immunochromatography, comprising the following components:
(1) a conjugate pad that carries a conjugate comprising a colloidal metal having a surface sensitized with a first antibody or an antigen, the surface of the colloidal metal being blocked with a component of non-biological origin and a component of biological origin; and
(2) an insoluble membrane that includes a second antibody that binds to a complex of the conjugate and a substance to be detected, the insoluble membrane being disposed downstream of the conjugate pad as the component (1).

15. The test strip according to claim 14, wherein the colloidal metal is colloidal platinum or colloidal gold.

16. The detection device, comprising: the test strip for immunochromatography according to claim 14 or 15; and a housing.

17. A manufacturing method for a conjugate comprising a colloidal metal sensitized with an antibody or an antigen, the manufacturing method comprising:
(a) sensitizing a colloidal metal with an antibody or an antigen to provide a conjugate;
(b) subjecting a surface of the colloidal metal to blocking treatment by bringing the conjugate into contact with a component of non-biological origin and a component of biological origin; and
(c) concentrating the conjugate subjected to the blocking treatment by centrifugation.

18. The manufacturing method for a conjugate according to claim 17, wherein the component of biological origin comprises one or more members selected from the group consisting of: BSA; casein; sericin; a polypeptide corresponding to amino acids 419 to 607 of an amino acid sequence of DnaK, which is a heat shock protein (HSP) of *Escherichia coli* origin; NEO PROTEIN SAVER; StartingBlock™ (PBS) Blocking Buffer; and Stabil Coat.

19. The manufacturing method for a conjugate according to claim 17 or 18, wherein the component of non-biological origin comprises one or more members selected from the group consisting of: a methacrylic acid polyoxyalkylene ester; NB4025 ;SN100 ;Stabil Guard; and Protein-Free Blocking Buffer.

20. The manufacturing method according to claim 17, wherein the step (b) comprises subjecting the surface of the colloidal metal to the blocking treatment by bringing the conjugate into contact with the component of non-biological origin, and then bringing the conjugate into contact with the component of biological origin.

21. The manufacturing method according to any one of claims 17 to 20, wherein the colloidal metal is colloidal platinum or colloidal gold.
